# EUROPEAN PATENT SPECIFICATION

(11) **EP 1 768 661 B1**
(45) Date of publication and mention of the grant of the patent: **20.08.2008**
(21) Application number: 05762789.5
(22) Date of filing: 22.06.2005
(51) Int. Cl.: A61K 31/335, A61P 17/02

(54) **USE OF TARGETED OXIDATIVE THERAPEUTIC FORMULATION IN TREATMENT OF BURNS**
VERWENDUNG EINER GEZIELTEN OXIDATIVEN THERAPEUTISCHEN FORMULIERUNG BEI DER BEHANDLUNG VON VERBRENNUNGEN
UTILISATION D'UNE PREPARATION THERAPEUTIQUE OXYDANTE CIBLEE DANS LE TRAITEMENT DES BRULURES

(30) Priority: 23.06.2004 US 582343 P
(43) Date of publication of application: 04.04.2007
(73) Proprietor: Hofmann, Robert F., Austin, TX 78746-2432 (US)
(72) Inventor: Hofmann, Robert F., Austin, TX 78746-2432 (US)
(74) Representative: Fisher, Adrian John
(86) International application number: PCT/US2005/022229
(87) International publication number: WO 2006/002302

(56) References cited:
- WO-A-02/078623
- US-A- 5 190 979
- US-A- 5 270 344

## Description

### BACKGROUND

The present invention relates to uses of a composition containing peroxidic species or oxidation products. More specifically, the invention relates to the use of a pharmaceutical composition or formulation which contains: peroxidic species or reaction products resulting from oxidation of an olefinic compound, in a liquid form or in a solution, by an oxygen-containing oxidizing agent; a penetrating solvent; a dye containing a chelated metal; and an aromatic redox compound for the preparation of the pharmaceutical formulation for resolving scar tissue, particularly scar tissue resulting from a burn.

After a severe burn or delayed wound repair with infection, scar tissue develops. Keloid and hypertrophic scars are marked by excessive collagen accumulation, secondary to neovascularization and fibroblast dysplasia. Keloid scars are an overgrowth of scar tissue. The scar grows beyond the site of the injury. Keloid scars are sometimes very nodular in nature, and they are often darker in color than surrounding skin. They occur when the body continues to produce tough, fibrous collagen after a wound has healed. Hypertrophic scars are red, thick and raised, but they differ from keloid scars in that they do not develop beyond the site of injury or incision. Hypertrophic scar formation is not a part of normal wound healing and can develop over time.

The potential use of antibody-targeted photolysis ("ATPL") in treating hypertrophic scars has been investigated. An immunoconjugate, consisting of a photosensitizer porphyrin (Sn-chlorin e6) linked to a monoclonal antibody that binds to human myofibroblasts (PR2D3), was prepared. When photoactivated, the complex produced singlet oxygen in close proximity to the target cell surface. The model used for the studies consisted of hypertrophic scar tissue implants in athymic mice. The hypertrophic implants increased 20-fold in volume over a period of 15 days. Four days after implantation immunoconjugate was injected directly into scar implants allowed to diffuse throughout for 24 hr before implants were illuminated with laser light at 630 nm (120 J/cm 2). ATPL treatment caused a significant reduction in total growth compared to the untreated controls (P < 0.05). No effect was observed when an irrelevant conjugate (anti-Pseudomonas aeruginosa) was used. Histological examination of the ATPL-treated implants 24 hr post-ATPL revealed the presence of a large number of lipid droplets, indicative of apoptosis with infiltration by mononuclear cells and neutrophils (Wolfort et al. 1996).

The assessment of deuteroporphyrin-hemin complex as an agent for the treatment of burn wounds infected with a multiple-drug resistant strain of *Staphylococcus aureus* has also been performed. The effect of the porphyrin on the survival of the infectious bacteria was first assayed in culture, and later tested as well in an infected burned animal model. The addition of deuteroporphyrin and hemin, separately or together (as a complex) to a growing culture of S. *aureus* was monitored during 8 hours. It was found that deuteroporphyrin alone was strongly bactericidal only after photosensitization. On the other hand, hemin alone was moderately bactericidal but light independent. A combination of both deuteroporphyrin and hemin was extremely potent even in the dark and did not require illumination to eradicate the bacteria. The in vivo experiments by application of the above porphyrins in combination to infected burn wounds in guinea pigs was an effective way to reduce dramatically the contaminating S. *aureus.* Reduction of more than 99% of the viable bacteria was noted after the porphyrin mixture was dropped on the eschar or injected into the eschar, an effect that lasted for up to 24 hours. The deuteroporphyrin-hemin complex is suggested as a new bactericidal treatment of S. *aureus* infected burns since it was found to be a potent and promising anti-Staphylococcal agent (Orenstein et al. 1997).

Keloid scars are currently the most debilitating long-term complication of the surviving burn or wound patient. One of the most troublesome aspects of keloid scars is their tendency to recur, sometimes requiring repeated treatment. At present, there is no routinely effective form of therapy, which currently includes cryo-destruction, steroids, radiation, and plastic surgery.

What is needed, therefore, is a means for reducing and resolving scar tissue, particularly scar tissue which resulted from a burn.

Ozone is a triatomic gas molecule and an allotropic form of oxygen. It may be obtained by means of an electrical discharge or intense ultraviolet light through pure oxygen. The popular misconception that ozone is a serious pollutant, the "free radical" theory of disease, and the antioxidant supplement market have comprehensibly prejudiced medical orthodoxy against its use as a treatment. Ozone therapy, however, is a misnomer. Ozone is an extremely reactive and unstable gas with mechanisms of action directly related to the byproducts that it generates through selective interaction with organic compounds present in the plasma and in the cellular membranes. The selective reaction of ozone with unsaturated olefins occurs at the carbon-carbon double bond, generating ozonides. Ozone is toxic by itself, and its reaction products, ozonides, are unstable and are not therapeutic by themselves.

Hydrogen peroxide (H₂O₂), discovered in 1818, is present in nature in trace amounts. Hydrogen peroxide is unstable and decomposes violently (or foams) when in direct contact with organic membranes and particulate matter. Light, agitation, heating, and iron all accelerate the rate of hydrogen peroxide decomposition in solution. Hydrogen peroxide by direct contact *ex vivo* kills microbes that have low levels of peroxide-destroying enzymes, such as the catalases. However, there is no bactericidal effect when hydrogen peroxide is infused into the blood of rabbits infected with peroxide-sensitive *E. coli.* Moreover, increasing the concentration of peroxide ex-vivo in rabbit or human blood containing *E. coli* produces no evidence of direct bactericidal activity. The lack of effect of high concentrations of hydrogen peroxide is directly related to the presence of the peroxide-destroying enzyme catalase in the host animal's blood. To have any effect, high concentrations of hydrogen peroxide have to be in contact with the bacteria for significant periods of time. Large amounts of hydrogen peroxide-destroying enzymes, such as catalase, normally present in the blood make it impossible for peroxide to exist in blood for more than a few seconds. Thus, hydrogen peroxide introduced into the blood stream by injection or infusion does not directly act as an extracellular germicide in blood or extracellular fluids.

However, hydrogen peroxide does participate in the bactericidal processes of activated macrophage cells. Activated macrophage cells are drawn to the site of infection, attach to the infectious organism, and ingest it. The killing of the organisms takes place inside the macrophage cell by hydrogen peroxide. Hydrogen peroxide oxidizes cellular chloride to the chlorine dioxide free radical, which destabilizes microbial membranes and, if persistent, induces apoptosis or cellular suicide. The critical therapeutic criteria for intracellular peroxidation are the selective delivery, absorption and activation of peroxidic carrier molecules into only diseased macrophages, which are believed to be incapable of upgraded catalase and glutathione reductase activity. Infused hydrogen peroxide is a generalized poison whereas targeted intracellular peroxidation is a selective therapeutic tool.

Macrophage cells play critical roles in immunity, bone calcification, vision, neural insulation (myelinization), detoxification, pump strength, and clearance of toxins from the body, depending upon their site of localization. The energy requirements of macrophages are met by intracellular structures called mitochondria. Mitochondria are often structurally associated with the microfilament internal cytoarchitecture. The folded internal layer of the mitochondria creates the high-energy molecule ATP, while the outer layer contains cytochromes and electron recycling molecules that generate peroxides. The outer layers of mitochondria are susceptible to toxic blockade or damage by endotoxins, mycotoxins, virally encoded toxins, drugs, heavy metals, and pesticides. When the peroxidation function of mitochondria is blocked, the filament architecture of the cell tends to cross-link, generating incorrect signals, incompetence, inappropriate replication, or premature cell death.

U.S. Patent No. 4,451,480 to De Villez teaches a composition and method for treating acne. The method includes topically treating the affected area with an ozonized material derived from ozonizing various fixed oil and unsaturated esters, alcohols, ethers and fatty acids.

U.S. Patent No. 4,591,602 to De Villez shows an ozonide of Jojoba used to control microbial infections.

U.S. Patent No. 4,983,637 to Herman discloses a method to parenterally treat local and systemic viral infections by administering ozonides of terpenes in a pharmaceutically acceptable carrier.

U.S. Patent No. 5,086,076 to Herman shows an antiviral composition containing a carrier and an ozonide of a terpene. The composition is suitable for systemic administration or local application.

U.S. Patent No. 5,126,376 to Herman describes a method to topically treat a viral infection in a mammal using an ozonide of a terpene in a carrier.

U.S. Patent No. 5,190,977 to Herman teaches an antiviral composition containing a non-aqueous carrier and an ozonide of a terpene suitable for systemic injection.

U.S. Patent No. 5,190,979 to Herman describes a method to parenterally treat a medical condition in a mammal using an ozonide of a terpene in a carrier.

U.S. Patent No. 5,260,342 to Herman teaches a method to parenterally treat viral infections in a mammal using an ozonide of a terpene in a carrier.

U.S. Patent No. 5,270,344 to Herman shows a method to treat a systemic disorder in a mammal by applying to the intestine of the mammal a trioxolane or a diperoxide derivative of an unsaturated hydrocarbon which derivative is prepared by ozonizing the unsaturated hydrocarbon dissolved in a non-polar solvent.

U.S. Patent No. 5,364,879 to Herman describes a composition for the treatment of a medical condition in a mammal, the composition contains a diperoxide or trioxolane derivative of a non-terpene unsaturated hydrocarbon which derivative is prepared by ozonizing below 35° C the unsaturated hydrocarbon in a carrier.

Despite the reports on the use of terpene ozonides for different medical indications, terpene ozonides display multiple deficiencies. For example, ozonides of monoterpene, such as myrcene and limonene, flamed out in the laboratory. Consequently, they are extremely dangerous to formulate or store.

Furthermore, ozonides of geraniol, a linear monoterpene alcohol, in water or in dimethylsulfoxide ("DMSO") did not show any clinical efficacy in three cases of viral Varicella Zoster (shingles) and two cases of Herpes Simplex dermatitis.
WO 02/078623 relates to compositions identical to the ones of the present application for the resolution of arterial graft scar formation and to keloid scar resulted of a vein graft donor side.

Thus, there is a need for a safe and effective pharmaceutical formulation or composition utilizing reaction products from the oxidation of an alkene compound. What is also needed is a method for stimulating mitochondrial activity in injured cells and resolving scar tissue such as that which results from a bum.

### SUMMARY

This invention is directed to the use of pharmaceutical formulations comprising peroxidic species or reaction products resulting from oxidation of an unsaturated organic compound, in a liquid form or in a solution, by an oxygen-containing oxidizing agent; a penetrating solvent; a chelated dye; and an aromatic redox compound. In one embodiment of the pharmaceutical formulation, the essential components include the peroxidic products formed by ozonolysis of an unsaturated alcohol, a stabilizing solvent, metalloporphyrin, and quinone for the preparation of a pharmaceutical formulation to resolve scar tissue, particularly scar tissue resulting from a burn.

The peroxidic species or reaction products are preferably formed through the reaction of an alkene and ozone. It is generally accepted that the reaction between an alkene and ozone proceeds by the Criegee mechanism. According to this mechanism, shown in Scheme 1 below, the initial step of the reaction is a 1,3-dipolar cycloaddition of ozone to the alkene to give a primary ozonide (a 1,2,3-trioxalane). The primary ozonide is unstable, and undergoes a 1,3-cycloreversion to a carbonyl compound and a carbonyl oxide. In the absence of other reagents or a nucleophilic solvent, this new 1,3-dipole enters into a second 1,3-dipolar cycloaddition to give the "normal" ozonide, a 1,2,4-trioxalane.

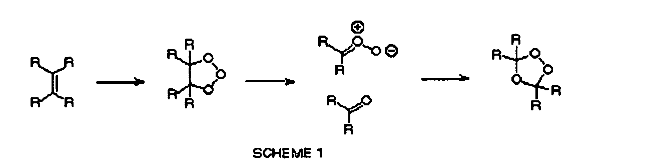

In a side reaction, the carbonyl oxide can enter into a dimerization to give a peroxidic dimer, the 1,2,4,5-tetraoxane, shown in Scheme 2 below.

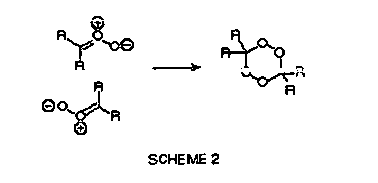

The carbonyl oxide is a strongly electrophilic species, and in the presence of nucleophilic species (e.g. alcohols or water), it undergoes facile nucleophilic addition to give a 1-alkoxyhydroperoxide, shown in Scheme 3 below. Under certain conditions, the 1-alkoxyhydroperoxide can undergo further reaction to give carboxylic acid derivatives.

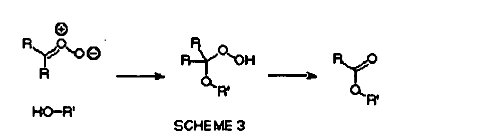

Again, not wanting to be bound by theory, it is believed that during the ozonolysis of the alcohol-containing alkene in the present invention, it is reasonable to expect that three major types of peroxidic products will be present: the normal ozonide, the carbonyl tetraoxane dimer, and the 1-alkoxyhydroperoxide. In the presence of water, some of these peroxidic products may also lead to the presence of organic peracids in the crude product mixture.

The present invention also involves the use of a penetrating solvent such as dimethylsulfoxide ("DMSO") to "stabilize" the initial products of the ozonolysis. Similarly, not wanting to be bound by any theory, it is believed that the stabilization is most likely a simple solvation phenomenon. However, DMSO is known to be a nucleophile in its own right. Its participation is also possible as a nucleophilic partner in stabilizing reactive species (for example, as dimethylsulfoxonium salts). The stabilized peroxidic molecule and the penetrating solvent of the current pharmaceutical formulation are made from components generally regarded as safe ("GRAS").

Another component of the pharmaceutical formulation is a chelated dye, such as a porphyrin. The propensity of metalloporphyrins to sensitize oxygen under photochemical excitation is well documented, as is the propensity of ferroporphyrins and copper porphyrins to bind oxygen-containing systems.

A further component of the pharmaceutical formulation is an aromatic redox compound, such as a quinone.

Although not wanting to be bound by any theory, it is postulated that the preferred pharmaceutical formulation is a combination of biochemical agents that induce recycling autocatalytic oxidation in infected or dysplastic macrophages. The pharmaceutical formulation stimulates targeted apoptosis (cell suicide) through unopposed peroxidation. Thus, the pharmaceutical formulation creates therapeutic effects in a number of seemingly disparate mitochondria-based macrophagic diseases.

### BRIEF DESCRIPTION OF DRAWINGS

Figure 1 is a photograph of a subject's burn injury 18 months after the injury occurred;

Figure 2 is a photograph of the subject's burn injury 30 days after an initial treatment with the pharmaceutical formulation, or a total of 6 intravenous treatments over the course of 4 weeks; and

Figure 3 is a photograph of the subject's burn injury 6 months after the initial treatment with the pharmaceutical formulation, or a total of 16 intravenous treatments with the pharmaceutical formulation over the course of 24 weeks.

### DETAILED DESCRIPTION OF PREFERRED EMBODIMENTS

The current invention pertains to the use of pharmaceutical formulations comprising peroxidic species or reaction products resulting from oxidation of an unsaturated organic compound, in a liquid form or in a solution, by an oxygen-containing oxidizing agent; a penetrating solvent; a chelated dye; and an aromatic redox compound, for the preparation of pharmaceutical formulations for resolve scar tissue and to treat individuals who have been burned. In one embodiment of the present invention, the essential components of the pharmaceutical formulation include the peroxidic products formed by ozonolysis of an unsaturated alcohol, a stabilizing solvent, metalloporphyrin, and quinone.

The unsaturated organic compound, which may also be an unsaturated olefinic hydrocarbon, of the pharmaceutical formulation can be an alkene without a hydroxyl group, or a hydroxyl-containing alkene. Preferably, the alkene has less than about 35 carbons. The alkene without a hydroxyl group may be an open-chain unsaturated hydrocarbon, a monocyclic unsaturated hydrocarbon, or a bicyclic unsaturated hydrocarbon. The hydroxyl-containing alkene can be an open-chain unsaturated alcohol, a monocyclic unsaturated alcohol, or a bicyclic unsaturated alcohol. The alkene may also be contained in a fixed oil, an ester, a fatty acid, or an ether.

Usable unsaturated olefinic hydrocarbons may be unsubstituted, substituted, cyclic or complexed alkenes, hydrazines, isoprenoids, steroids, quinolines, carotenoids, tocopherols, prenylated proteins, or unsaturated fats. The preferred unsaturated hydrocarbons for this invention are alkenes and isoprenoids.

Isoprenoids are found primarily in plants as constituents of essential oils. While many isoprenoids are hydrocarbons, oxygen-containing isoprenoids also occur such as alcohols, aldehydes, and ketones. In a formal sense, the building block of isoprenoid hydrocarbons may be envisaged as the hydrocarbon isoprene, CH₂=C(CH₃)-CH=CH₂, although it is known that isoprene itself is an end-produet of isoprenoid biosynthesis and not an intermediate. Isoprenoid hydrocarbons are categorized by the number of isoprene (C₅H₈) units they contain. Thus, monoterpenes have 2, sesquiterpenes have 3, diterpenes have 4, sesterterpenes have 5, triterpenes have 6, and tetraterpenes have 8 isoprene units, respectively. Tetraterpenes are much more commonly known as carotenoids.

Limonene and pinene are examples of a monoterpene. Farnesol and nerolidol are examples of a sesquiterpene alcohol. Vitamin A₁ and phytol are examples of a diterpene alcohol while squalene is an example of a triterpene. Provitamin A₁, known as carotene, is an example of a tetraterpene. Geraniol, a monoterpene alcohol, is liquid in both its oxygen bound and normal states and is safe to living cells.

Preferred unsaturated hydrocarbons for the pharmaceutical formulation include alkene isoprenoids, such as myricene, citrillene, citral, pinene, or limonene. Preferred unsaturated hydrocarbons also include linear isoprenoid alcohols with two to four repeating isoprene groups in a linear chain, such as terpineol, citronellol, nerol, phytol, menthol, geraniol, geranylgeraniol, linalool, or farnesol.

The unsaturated organic compound may be linear, branched, cyclic, spiral, or complexed with other molecules in its configuration. The unsaturated organic compound may naturally exist in a gaseous liquid or solid state prior to binding with the oxidizing agent.

An open-chain unsaturated hydrocarbon can be: CₙH₂ₙ, one double bond, n=2-20; CₙH₂ₙ₋₂, two double bonds, n=4-20; CₙH₂ₙ₋₄, three double bonds, n=6-20; CₙH₂ₙ₋₆, four double bonds, n=8-20; C₂₅H₄₀, sesterterpene hydrocarbon; or C₃₀H₄₈, triterpene hydrocarbon.

A monocyclic unsaturated hydrocarbon can be: CₙH₂ₙ₋₂, one double bond and one ring, n=3-20; CₙH₂ₙ₋₄, two double bonds and one ring, n=5-20; CₙH₂ₙ₋₆, three double bonds and one ring, n=7-20; C₂₅H₄₀, sesterterpene hydrocarbon; or C₃₀H₄₈, triterpene hydrocarbon.

A bicyclic unsaturated hydrocarbon can be: CₙH₂ₙ₋₄, one double bond and two rings, n=4-20; CₙH₂ₙ₋₆, two double bonds and two rings, n=6-20; C₂₅H₄₀, sesterterpene hydrocarbon; or C₃₀H₄₈, tnterpene hydrocarbons.

An open-chain unsaturated alcohol can be: CₙH₂ₙOₘ, one double bond, n=3-20, m=1-4; CₙH₂ₙ₋₂Oₘ, two double bonds, n=5-20, m=1-4; CₙH₂ₙ₋₄Oₘ, three double bonds, n=7-20, m=1-4; CₙH₂ₙ₋₆Oₘ, four double bonds, n=9-20, m=1-4; C₂₅H₄₀Oₘ, m=1-4, sesterterpene alcohols; or C₃₀H₄₈Oₘ, m=1-4, triterpene alcohols.

A monocyclic unsaturated alcohol can be: CₙH₂ₙ₋₂Oₘ, one double bond and one ring, n=3-20, m=1-4; CₙH₂ₙ₋₄Oₘ, two double bonds and one ring, n=5-20, m=1-4; CₙH₂ₙ₋₆Oₘ, three double bonds + one ring, n=7-20, m=1-4; C₂₅H₄₀Oₘ, m=1-4, sesterterpene alcohols; or C₃₀H₄₈Oₘ, m=1-4, triterpene alcohols.

A bicyclic unsaturate alcohol can be: CₙH₂ₙ₋₄Oₘ, one double bond and two rings, n=5-20, m=1-4; CₙH₂ₙ₋₆Oₘ, two double bonds and two rings, n=7-20, m=1-4; C₂₅H₄₀Oₘ, m=1-4, sesterterpene alcohols; or C₃₀H₄₈Oₘ, m=1-4, triterpene alcohols.

Based on the total weight of the pharmaceutical formulation, the alkene can vary from about 0.001 % to about 30%, preferably from about 0.1 % to about 5.0%, and more preferably from about 0.5% to about 3.0%.

The oxygen-containing oxidizing agent of the pharmaceutical formulation, which oxidizes the unsaturated hydrocarbon, may be singlet oxygen, oxygen in its triplet state, superoxide anion, ozone, periodate, hydroxyl radical, hydrogen peroxide, alkyl peroxide, carbamyl peroxide, benzoyl peroxide, or oxygen bound to a transition element, such as molybdenum (e.g. MoO₅).

The preferred method to bind "activated oxygen" to intact an isoprenoid alcohol, such as geraniol, is by ozonation at temperatures between 0-20°C in the dark in the absence of water or polar solvent. The geraniol "ozonides" are then dissolved and stabilized in 100% DMSO in the dark to prevent premature breakdown of the products. Although not wanting to be bound by any theory, it is believed that the catalytic breakdown of the tetraoxane peroxidic dimer byproduct of geraniol ozonation, which is not an ozonide, occurs inside of cells in the presence of superoxide anion. The final reactive therapeutic agents released are hydrogen peroxide and acetic acid.

The pharmaceutical formulation also utilizes a penetrating solvent. The penetrating solvent, which stablizes the oxygen-bound unsaturated hydrocarbon, may be an emollient, a liquid, a liposome, a micelle membrane, or a vapor. Usable penetrating solvents include aqueous solution, fats, sterols, lecithins, phosphatides, ethanol, propylene glycol, methylsulfonylmethane, polyvinylpyrrolidone, pH-buffered saline, and dimethylsulfoxide ("DMSO"). The preferred penetrating solvents include DMSO, polyvinylpyrrolidone, and pH-buffered saline. The most preferred penetrating solvent is DMSO.

Based on the total weight of the pharmaceutical formulation, the penetrating solvent can vary from about 50% to about 99%, preferably from about 90% to about 98%, and more preferably from about 95% to about 98%.

The "stabilized" peroxidic molecule and its penetrating solvent have been made from components currently used in production regulated by the Food and Drug Administration ("FDA"). These ingredients are the subject of Drug Master Files, Drug Monographs, are found in the USP/NF, or are Generally Recognized As Safe ("GRAS").

Another component of the pharmaceutical formulation is a chelated dye. The dye preferably contains a chelated divalent or trivalent metal, such as iron, copper, manganese tin, magnesium, or strontium. The preferred chelated metal is iron. The propensity of chelated dyes such as metalloporphyrins to sensitize oxygen under photochemical excitation is well documented, as is the propensity of ferroporphyrins and copper porphyrins to bind oxygen-containing systems. Usable dyes include natural or synthetic dyes. Examples of these dyes include porphyrins, rose bengal, chlorophyllins, hemins, porphins, corrins, texaphrins, methylene blue, hematoxylin, eosin, erythrosin, flavinoids, lactoflavin, anthracene dyes, hypericin, methylcholanthrene, neutral red, phthalocyanine, fluorescein, eumelanin, and pheomelanin. Preferred dyes can be any natural or synthetic porphyrin, hematoporphyrin, chlorophyllin, rose bengal, their respective congeners, or a mixture thereof. The most preferred dyes are naturally occurring porphyrins, such as hematoporphyrin, and rose bengal. The dye may be responsive to photon, laser, ionizing radiation, phonon, electrical cardiac pulse, electroporation, magnetic pulse, or continuous flow excitation.

Based on the total weight of the pharmaceutical formulation or composition, the dye can vary from about 0.1% to about 30%, preferably from about 0.5% to about 5%, and more preferably from about 0.8% to about 1.5%.

A further component of the pharmaceutical formulation is an aromatic redox compound, such as a quinone. The aromatic redox compound may be any substituted or unsubstituted benzoquinone, naphthoquinone, or anthroquinone. Preferred aromatic redox compounds include benzoquinone, methyl-benzoquinone, naphthoquinone, and methyl-naphthoquinone. The most preferred aromatic redox compound is methyl-naphthoquinone.

Based on the total weight of the pharmaceutical formulation, the aromatic redox compound can vary from about 0.01% to about 20.0%, preferably from about 0.1% to about 10%, and more preferably from about 0.1% to about 0.5%.

The pharmaceutical formulation is also preferably activated by an energy source or an electron donor. Useful electron donors include an electrical current, ascorbate or ascorbic acid, NADH, NADPH and germanium sesquioxide. Preferred electron donors include ascorbate and germanium sesquioxide. The most preferred electron donor is ascorbic acid in any salt form.

Based on the total weight of the pharmaceutical formulation, the electron donor can vary from about 0.01% to about 20%, preferably from about 1% to about 10%, and more preferably from about 1% to about 5%.

In order to obtain a biological effect *in vivo,* the pharmaceutical formulation is preferably infused as an ozonolysis-generated peroxidic product of an unsaturated hydrocarbon, rather than an ozonide, in conjunction with a superoxide generating chelated dye and an aromatic quinone. The unsaturated hydrocarbon product, or peroxidic dimer molecule, should be stabilized in a non-aqueous stabilizing solvent and should be capable of penetrating lipid membranes.

Researchers of energetically activated dye therapy have long known that the superoxide generating dye and the aromatic redox compound preferentially absorb into infected and dysplastic cells, which are typically also catalase deficient. Without wanting to be bound by theory, the catalase-induced destruction of peroxide should be overwhelmed in the target cells either naturally or by the pharmaceutical formulation. The peroxidic dimer should also be activated by the superoxide generating dye, initiating electron donation to the dimer and causing the release of hydrogen peroxide and acetic acid intracellularly. The electronic activation of the dye does not always require light, but rather may occur through small electrical pulses provided by, for example, a heart pulse. The peroxidation reaction within the infected macrophage then tends to destroy the prenylated protein linkage of microtubules within the cell, to destroy the infecting toxin, or to induce apoptosis of the macrophage host cell.

The pharmaceutical formulation is a combination of stable ingredients. These ingredients may preferably be stored as dry solid ingredients and liquid ingredients in separate containers, which are then mixed at the site of use. The dry solid ingredients preferably comprise the chelated dye and the aromatic redox compound. The liquid ingredients preferably comprise the peroxidic species or reaction products resulting from oxidation of the unsaturated hydrocarbon by the oxygen-containing active agent, along with the penetrating solvent. Administration is preferably intravenously. The reconstituted product preferably may be administered intravenously as a concentrate diluted in saline. Topical, ocular, intraperitoneal, rectal and intrathecal deliveries are also possible routes for administration. Intramuscular injection is not preferred, as it has a tendency to produce local irritation.

Administration of the pharmaceutical formulation *in vivo* is effective in resolving scar tissue, particularly scar tissue resulting from a burn, and in treating individuals who have been burned.

### EXAMPLE 1. OZONOLYSIS OF AN UNSATURATED HYDROCARBON

Ozonolysis of an alkene may be carried out either in a solvent or neat. In either case, the cooling of the reaction mixture is critical in avoiding explosive decomposition of the peroxidic products of the reaction.

The following general procedure is typical for the ozonolysis of a liquid alkene.

A 1-liter flask fitted with a magnetic stirrer is charged with the alkene (2 moles), and the apparatus is weighed. The flask is surrounded by a cooling bath (ice-water or ice-salt). Once the contents are cooled below 5° C, stirring is begun and a stream of ozone in dry oxygen (typically 3% ozone) is passed through the mixture. It is advantageous to disperse the ozonated oxygen through a glass frit, but this is not necessary for a stirred solution. Periodically, the gas stream is stopped, and the reaction flask is weighed or the reaction mixture is sampled. The gas stream is then re-started.

Once the mass of the reaction flask shows sufficient weight gain, or once the proton magnetic resonance ("H¹ NMR") spectrum of the reaction mixture shows the desired reduction in the intensity of the olefinic proton resonances (usually about 50%), the gas flow is stopped.

The ozonolysis may be carried out as above, substituting a solution of the alkene in a solvent non-reactive towards ozone such as saturated hydrocarbons or chlorinated hydrocarbons. The ozonolysis may also be carried out as above, with or without solvent, substituting an alkenol for the alkene without affecting the reaction in any substantive manner.

The reaction mixture is then poured slowly into the cooled penetrating solvent.

### EXAMPLE 2. PREPARATION OF THE PHARMACEUTICAL FORMULATION

A preferred pharmaceutical formulation of the present invention was prepared as follows:
(1) Sparging an ozone/pure oxygen gas mixture of 120 mg/L up through an alkadiene alcohol, 3,7-dimethyl-2,6-octadien-1-ol (geraniol), at I Liter of gas per hour;
(2) Maintaining the temperature of the reaction around 5°C;
(3) Removing small aliquots of reaction product hourly and measuring by H' NMR the formation of the peroxidic species or reaction products;
(4) Stopping the reaction when more than about 50% of the available unsaturated bonds have been reacted;
(5) Diluting the product mixture with dimethylsulfoxide (1:10) to give a solution or dispersion;
(6) Prior to use in the target biological system, a mixture of hematoporphyrin, rose bengal, and methyl-naphthoquinone dry powders was added to the solution or dispersion in sufficient quantity to create a concentration of 20 micromolar of each component dispersed therein when delivered to the target biological system by saline intravenous infusion. Optionally, ascorbate could be added to the formulation prior to use.

### EXAMPLE 3. EXAMPLES OF THE PHARMACEUTICAL FORMULATION

Two preferred formulations are as follows:
**A.**

| **WEIGHT %** | **INGREDIENT** |
|---|---|
| 0.54* | Tetraoxane dimer of acetal peroxide from ozonation of geraniol |
| 98.00 | DMSO |
| 0.83 | Hematoporphyrin |
| 0.24 | Methylnaphthoquinone |
| 0.39 | Rose Bengal |

| | |
|---|---|
| *Determined by mass spectroscopy. | |

**B.**

| **WEIGHT %** | **INGREDIENT** |
|---|---|
| 0.54* | Tetraoxane dimer of acetal peroxide from ozonation of geraniol |
| 98.00 | DMSO |
| 0.83 | Hematoporphyrin |
| 0.24 | Methylnaphthoquinone |
| 0.39 | Chlorophyllin Sodium-Copper Salt |

| | |
|---|---|
| *Determined by mass spectroscopy. | |

### EXAMPLE 4. SCAR TISSUE REDUCTION IN VIVO

The subject was burned in a residential accident resulting from spontaneous combustion of bedtime clothing while opening and removing food from a heated oven. Severity of the burns ranged from first to third degree, covering an area from the patient's navel to chin. Heat from the burn was extreme enough to cause the extrusion of breast implants. Patient was hospitalized and treated m a professional burn center in Arkansas, and over the course of 18 months after the burn was admitted for five incidents of sepsis. During this 18 month period, prior to RACO treatment, pronounced keloid scarring had developed at several sites throughout the bum field.

Intravenous infusion of Formulation B (diluted 1 cc into 100 cc of normal saline) was accomplished over 20 minutes, for a total of six treatments over a 30 day period. No proximate or late adverse effects were noted. Subsequent treatments totaled 16 treatments over a six month period.

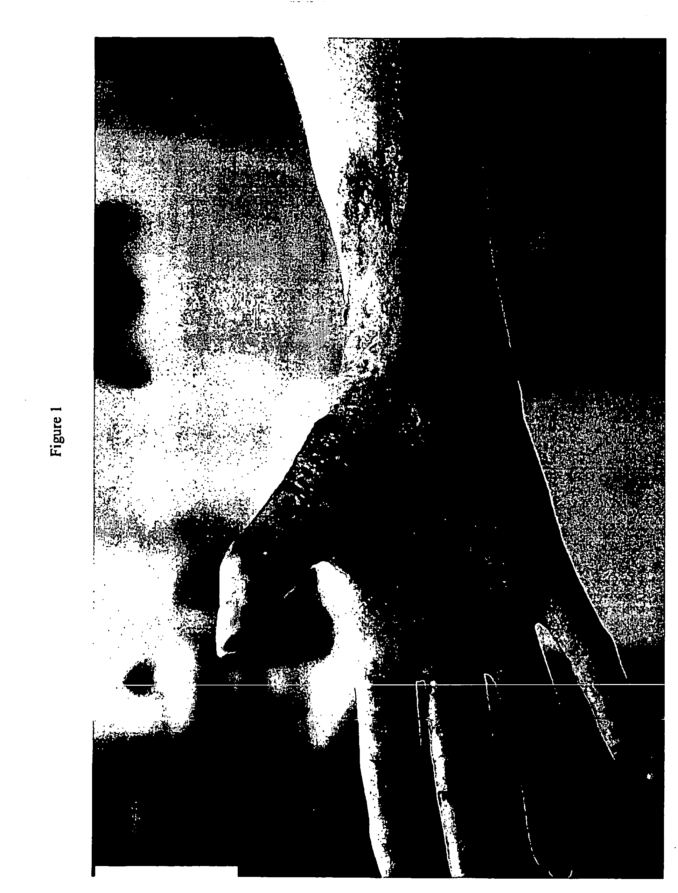

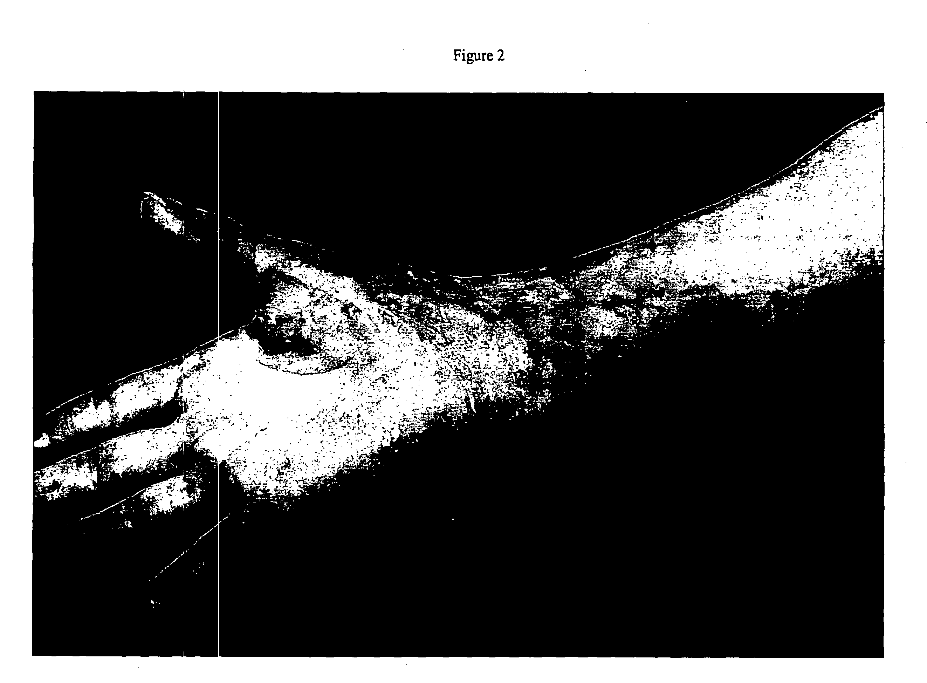

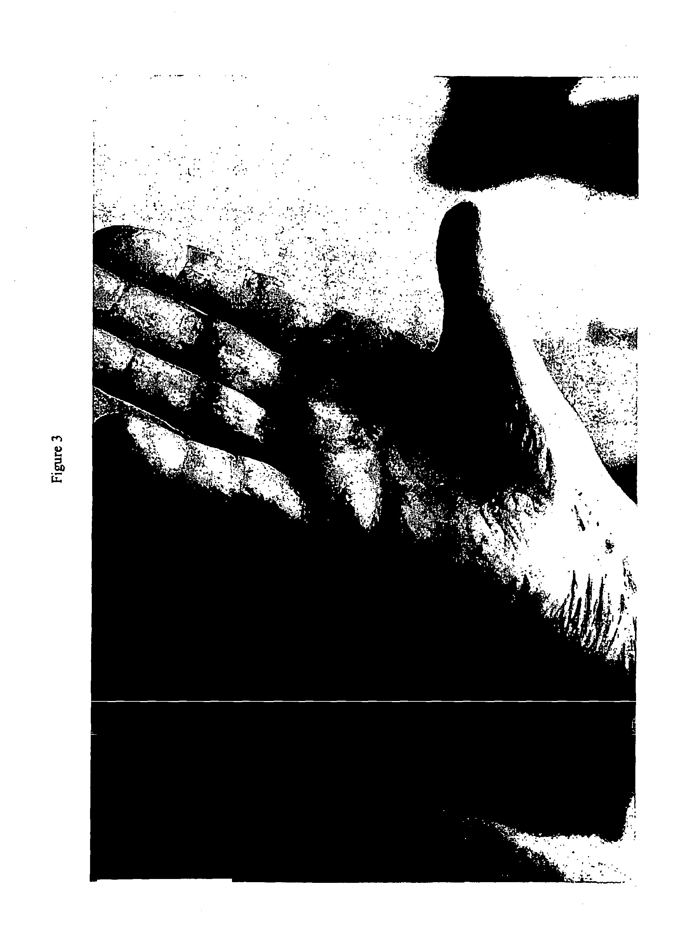

Photographic documentation by the Arkansas burn center showed evidence of rapid resolution of the keloid scar tissue. Figure 1 shows the patient's burn injury 18 months after it occurred. Figure 2 shows the injury after the first six treatments over a 30 day period. Figure 3 shows the injury after the subsequent 16 treatments over a 6 month period. The patient reported the scars reddened, hardened and sloughed off over time. Smooth but contracted dermal healing with intact surface epithelium was maintained for four years, allowing successful secondary plastic surgical reconstructions.

### REFERENCES CITED

U.S. Patents U.S. Patent No. 4,451,480 to DeVillez
   U.S. Patent No. 4,591,602 to DeVillez
   U.S. Patent No. 4,983,637 to Herman
   U.S. Patent No. 5,086,076 to Herman
   U.S. Patent No. 5,126,376 to Herman
   U.S. Patent No. 5,190,977 to Herman
   U.S. Patent No. 5,190,979 to Herman
   U.S. Patent No. 5,260,342 to Herman
   U.S. Patent No. 5,270,344 to Herman
   U.S. Patent No. 5,364,879 to Herman
   WO 02 /078623
**Other Publications**
   Orenstein, A., Klein, D., et al., The use of porphyrins for eradication of Staphylococcus aureus in burn wound infections. FEMS Immunol Med Microbiol vol. 19(4), pp. 307-44, Dec 1997.
   Wolfort, S.F., Reiken, S.R., et al., Control of hypertrophic scar growth using antibody-targeted photolysis. J Surg Res vol. 62(1), pp. 17 - 22, Apr 1996.

## Claims

1. Use of peroxidic species or reaction products resulting from oxidation of menthol or an alkene by an oxygen-containing oxidizing agent, wherein the alkene comprises terpineol, citronellol, nerol, linalool, phytol, geraniol, perillyl alcohol, menthol, geranylgeraniol or farnesol, and wherein the peroxidic species or reaction products resulting from oxidation of menthol or the alkene is from about 0.001% to about 30% by weight of the pharmaceutical formulation;
a penetrating solvent, wherein the penetrating solvent comprises dimethylsulfoxide, sterol, lecithin, propylene glycol, or methylsulfonylmethane, and wherein the penetrating solvent is from about 50% to about 99% by weight of the pharmaceutical formulation;
a dye containing a chelated divalent or trivalent metal, wherein the dye comprises porphyrin, rose bengal, chlorophyllin, hemin, corrins, texaphrin, methylene blue, hematoxylin, cosin, erythrosin, lactoflavin, anthracene dye, hypericin, methylcholanthrene, neutral red, phthalocyanine, fluorescein, eumelanin, or pheomelanin, and wherein the dye is from about 0.1% to about 30% by weight of the pharmaceutical formulation; and
an aromatic redox compound, wherein the redox compound comprises substituted or unsubstituted benzoquinone, naphthoquinone, or anthroquinone, and wherein the aromatic redox compound is from about 0.01% to about 20% by weight of the pharmaceutical formulation
for the manufacture of a pharmaceutical formulation for resolving gear tissue resulting from a burn.

2. The use of claim 1, wherein the alkene is in a liquid form, in a solution, or in a dispersion.

3. The use of claim 1, wherein the alkene is contained in a fixed oil, an ester, a fatty acid, or an ether.

4. The use of claim 1, wherein the oxygen-containing oxidizing agent comprises singlet oxygen, oxygen in its triplet state, superoxide anion, periodate, hydroxyl radical, hydrogen peroxide, alkyl peroxide, carbamyl peroxide, benzoyl peroxide, or oxygen bound to a transition element.

5. The use of claim 1, wherein the oxygen-containing oxidizing agent is predominantly ozone.

6. The use of claim 1, wherein the penetrating solvent is a liquid, micelle membrane, liposome, emollient, or vapor.

7. The use of claim 1, wherein the penetrating solvent is dimethylsulfoxide ("DMSO").

8. The use of claim 1, wherein the dye comprises porphyrin, rose bengal, or a mixture thereof.

9. The use of claim 1, wherein the metal comprises iron.

10. The use of claim 1, wherein the metal comprises copper, manganese, tin, magnesium, or strontium.

11. The use of claim 1, further comprising an electron donor.

12. The use of claim 11, wherein the electron donor comprises ascorbic acid or a pharmaceutical salt thereof.

13. Use of
peroxidic species or reaction products resulting from oxidation of geraniol by a mixture of ozone and oxygen;
dimethylsulfoxide ("DMSO");
a dye containing a chelated divalent or trivalent metal, wherein the dye comprises a mixture of hematoporphyrin and rose bengal or a mixture of hematoporphyrin and chlorophyllin; and methylnaphthoquinone, for the manufacture of a pharmaceutical formulation for resolving scar tissue resulting from a burn.

14. The use of claim B, wherein the scar tissue resulted from a burn to the patient.

15. Use of
peroxidic species or reaction products resulting from oxidation of menthol or an alkene by an oxygen-containing oxidizing agent, wherein the alkene comprises terpineol, citronellol, nerol, linalool, phytol, geraniol, perillyl alcohol, menthol, geranylgeraniol or farnesol, and wherein the peroxidic species or reaction products resulting from oxidation of menthol or the alkene is from about 0.001% to about 30% by weight of the pharmaceutical formulation;
a penetrating solvent, wherein the penetrating solvent comprises dimethylsulfoxide, sterol, lecithin, propylene, glycol, or methylsulfonylmethane, and wherein the penetrating solvent is from about 50% to about 99% by weight of the pharmaceutical
a dye containing a chelated divalent or trivalent metal, wherein the dye comprises porphyrin, rose bengal, chlorophyllin, hemin, corrins, texaphrin, methylene blue, hematoxylin, eosin, erythrosin, lactoflavin, anthracene dye, hypericin, methylcholanthrene, neutral red, phthalocyanine, fluorescein, eumelanin, or pheomelanin, and wherein the dye is from about 0.1% to about 30% by weight of the pharmaceutical formulation; and
an aromatic redox compound, wherein the redox compound comprises substituted or unsubstituted benzoquinone, naphthoquinone, or anthroquinone, and wherein the aromatic redox compound is from about 0.01% to about 20% by weight of the pharmaceutical formulation, for the manufacture of a pharmaceutical formulation for treating an injury resulting from a burn.

16. The use of claim 1, wherein the alkene is in a liquid form, in a solution, or in a dispersion.

17. The use of claim 1, wherein the alkene is contained in a fixed oil, an ester, a fatty acid, or an ether.

18. The use of claim 1, wherein the oxygen-containing oxidizing agent comprises singlet oxygen, oxygen in its triplet state, superoxide anion, periodate, hydroxyl radical, hydrogen peroxide, alkyl peroxide, carbamyl peroxide, benzoyl peroxide, or oxygen bound to a transition element.

19. The use of claim 1, wherein the oxygen-containing oxidizing agent comprises ozone.

20. The use of claim 1, wherein the penetrating solvent is a liquid, micelle membrane, liposome, emollient, or vapor.

21. The use of claim 1, wherein the penetrating solvent is dimethylsulfoxide ("DMSO")

22. The use of claim 1, wherein the dye comprises porphyrin, rose bengal, or a mixture thereof.

23. The use of claim 1, wherein the metal comprises iron.

24. The use of claim 1, wherein the metal comprises copper, manganese, tin, magnesium, or strontium.

25. The use of claim 1, further comprising an electron donor.

26. The use of claim 11, wherein the electron donor comprises ascorbic acid or a pharmaceutical salt thereof.

27. Use of
peroxidic species or reaction products resulting from oxidation of geraniol by a mixture of ozone and oxygen; dimethylsulfoxide ("DMSO");
a dye containing a chelated divalent or trivalent metal, wherein the dye comprises a mixture of hematoporphyrin and rose bengal or a mixture of hematoporphyrin and chlorophyllin; and methylnaphthoquinone, for the manufacture of a pharmaceutical formulation for treating an injury resulting from a burn.

## Patentansprüche

1. Verwendung von peroxidischen Spezies oder Reaktionsprodukten, die von der Oxidation von Menthol oder eines Alkens mit einem Sauerstoff enthaltenden Oxidationsmittel herrühren, wobei das Alken Terpineol, Citronellol, Nerol, Linalool, Phytol, Geraniol, Perillaalkohol, Menthol, Geranylgeraniol oder Farnesol umfasst und wobei die peroxidischen Spezies oder Reaktionsprodukte, die von der Oxidation von Menthol oder des Alkens herrühren, etwa 0,001 Gew.-% bis etwa 30 Gew.-% der pharmazeutischen Formulierung ausmachen;
einem penetrierenden Lösemittel, wobei das penetrierende Lösemittel Dimethylsulfoxid, Sterol, Lecithin, Propylenglykol oder Methylsulfonylmethan umfasst und wobei das penetrierende Lösemittel etwa 50 Gew.-% bis etwa 99 Gew.-% der pharmazeutischen Formulierung ausmacht;
einem Farbstoff, der ein chelatisiertes zweiwertiges oder dreiwertiges Metall enthalt, wobei der Farbstoff Porphyrin, Bengalrosa, Chlorophyllin, Hamin, Corrine, Texaphrin, Methylenblau, Hämatoxylin, Eosin, Erythrosin, Lactoflavin, Anthracenfarbstoff, Hypericin, Methylcholanthren, Neutralrot, Phthalocyanin, Fluorescein, Eumelanin oder Phäomelanin umfasst und wobei der Farbstoff etwa 0,1 Gew.-% bis etwa 30 Gew.-% der pharmazeutischen Formulierung ausmacht; und
einer aromatischen Redoxverbindung, wobei die Redoxverbindung substituiertes oder unsubstituiertes Benzochinon, Naphthochinon oder Anthrachinon umfasst und wobei die aromatische Redoxverbindung etwa 0,01 Gew.-% bis etwa 20 Gew.-% der pharmazeutischen Formulierung ausmacht;
zur Herstellung einer pharmazeutischen Formulierung zum Auflösen von Narbengewebe, das von einer Verbrennung herruhrt.

2. Verwendung nach Anspruch 1, wobei das Alken in einer flüssigen Form, in einer Lösung oder in einer Dispersion vorliegt.

3. Verwendung nach Anspruch 1, wobei das Alken in einem nicht-flüssigen Öl, einem Ester, einer Fettsaure oder einem Ether enthalten ist.

4. Verwendung nach Anspruch 1, wobei das Sauerstoff enthaltende Oxidationsmittel Singulettsauerstoff, Sauerstoff in seinem Triplettzustand, Superoxidanion, Periodat, Hydroxylradikal, Wasserstoffperoxid, Alkylperoxid, Carbamylperoxid, Benzoylperoxid oder Sauerstoff, der an ein Übergangselement gebunden ist, umfasst.

5. Verwendung nach Anspruch 1, wobei das Sauerstoff enthaltende Oxidationsmittel überwiegend Ozon ist.

6. Verwendung nach Anspruch 1, wobei das penetrierende Lösemittel eine Flüssigkeit, Mizellenmembran, Liposom, Emolliens oder Dampf ist.

7. Verwendung nach Anspruch 1, wobei das penetrierende Lösemittel Dimethylsulfoxid ("DMSO") ist.

8. Verwendung nach Anspruch 1, wobei der Farbstoff Porphyrin, Bengalrosa oder ein Gemisch davon umfasst.

9. Verwendung nach Anspruch 1, wobei das Metall Eisen umfasst.

10. Verwendung nach Anspruch 1, wobei das Metall Kupfer, Mangan, Zinn, Magnesium oder Strontium umfasst.

11. Verwendung nach Anspruch 1, ferner umfassend einen Elektronendonor.

12. Verwendung nach Anspruch 11, wobei der Elektronendonor Ascorbinsäure oder ein pharmazeutisches Salz davon umfasst.

13. Verwendung von
peroxidischen Spezies oder Reaktionsprodukten, die von der Oxidation von Geraniol mit einem Gemisch aus Ozon und Sauerstoff herrühren;
Dimethylsulfoxid ("DMSO");
einem Farbstoff, der ein chelatisiertes zweiwertiges oder dreiwertiges Metall enthält, wobei der Farbstoff ein Gemisch aus Hämatoporphyrin und Bengalrosa oder ein Gemisch aus Hämatoporphyrin und Chlorophyllin umfasst; und
Methylnaphthochinon;
zur Herstellung einer pharmazeutischen Formulierung zum Auflösen von Narbengewebe, das von einer Verbrennung herruhrt.

14. Verwendung nach Anspruch 13, wobei das Narbengewebe von einer Verbrennung an einem Patienten herrührte.

15. Verwendung von
peroxidischen Spezies oder Reaktionsprodukten, die von der Oxidation von Menthol oder eines Alkens mit einem Sauerstoff enthaltenden Oxidationsmittel herruhren, wobei das Alken Terpineol, Citronellol, Nerol, Linalool, Phytol, Geraniol, Perillaalkohol, Menthol, Geranylgeraniol oder Farnesol umfasst und wobei die peroxidischen Spezies oder Reaktionsprodukte, die von der Oxidation von Menthol oder des Alkens herruhren, etwa 0,001 Gew.-% bis etwa 30 Gew.-% der pharmazeutischen Formulierung ausmachen;
einem penetrierenden Lösemittel, wobei das penetrierende Lösemittel Dimethylsulfoxid, Sterol, Lecithin, Propylenglykol oder Methylsulfonylmethan umfasst und wobei das penetrierende Lösemittel etwa 50 Gew.-% bis etwa 99 Gew.-% der pharmazeutischen Formulierung ausmacht;
einem Farbstoff, der ein chelatisiertes zweiwertiges oder dreiwertiges Metall enthalt, wobei der Farbstoff Porphyrin, Bengalrosa, Chlorophyllin, Hämin, Corrine, Texaphrin, Methylenblau, Hamatoxylin, Eosin, Erythrosin, Lactoflavin, Anthracenfarbstoff, Hypericin, Methylcholanthren, Neutralrot, Phthalocyanin, Fluorescein, Eumelanin oder Phäomelanin umfasst und wobei der Farbstoff etwa 0,1 Gew.-% bis etwa 30 Gew.-% der pharmazeutischen Formulierung ausmacht; und
einer aromatischen Redoxverbindung, wobei die Redoxverbindung substituiertes oder unsubstituiertes Benzochinon, Naphthochinon oder Anthrachinon umfasst und wobei die aromatische Redoxverbindung etwa 0,01 Gew.-% bis etwa 20 Gew.-% der pharmazeutischen Formulierung ausmacht;
zur Herstellung eines Arzneimittels zum Behandeln einer Verletzung, die von einer Verbrennung herrührt.

16. Verwendung nach Anspruch 1, wobei das Alken in einer flüssigen Form, in einer Lösung oder in einer Dispersion vorliegt.

17. Verwendung nach Anspruch 1, wobei das Alken in einem nicht-flüssigen Öl, einem Ester, einer Fettsäure oder einem Ether enthalten ist.

18. Verwendung nach Anspruch 1, wobei das Sauerstoff enthaltende Oxidationsmittel Singulettsauerstoff, Sauerstoff in seinem Triplettzustand, Superoxidanion, Periodat, Hydroxylradikal, Wasserstoffperoxid, Alkylperoxid, Carbamylperoxid, Benzoylperoxid oder Sauerstoff, der an ein Übergangselement gebunden ist, umfasst.

19. Verwendung nach Anspruch 1, wobei das Sauerstoff enthaltende Oxidationsmittel Ozon umfasst.

20. Verwendung nach Anspruch 1, wobei das penetrierende Lösemittel eine Flüssigkeit, Mizellenmembran, Liposom, Emolliens oder Dampf ist.

21. Verwendung nach Anspruch 1, wobei das penetrierende Lösemittel Dimethylsulfoxid ("DMSO") ist.

22. Verwendung nach Anspruch 1, wobei der Farbstoff Porphyrin, Bengalrosa oder ein Gemisch davon umfasst.

23. Verwendung nach Anspruch 1, wobei das Metall Eisen umfasst.

24. Verwendung nach Anspruch 1, wobei das Metall Kupfer, Mangan, Zinn, Magnesium oder Strontium umfasst.

25. Verwendung nach Anspruch 1, ferner umfassend einen Elektronendonor.

26. Verwendung nach Anspruch 11, wobei der Elektronendonor Ascorbinsäure oder ein pharmazeutisches Salz davon umfasst.

27. Verwendung von
peroxidischen Spezies oder Reaktionsprodukten, die von der Oxidation von Geraniol mit einem Gemisch aus Ozon und Sauerstoff herrühren;
Dimethylsulfoxid ("DMSO");
einem Farbstoff, der ein chelatisiertes zweiwertiges oder dreiwertiges Metall enthält, wobei der Farbstoff ein Gemisch aus Hämatoporphyrin und Bengalrosa oder ein Gemisch aus Hamatoporphyrin und Chlorophyllin umfasst; und
Methylnaphthochinon;
zur Herstellung einer pharmazeutischen Formulierung zum Behandeln einer Verletzung, die von einer Verbrennung herrührt.

## Revendications

1. Utilisation d'espèces peroxydiques ou de produits de réaction provenant de l'oxydation du menthol ou d'un alcène par un agent d'oxydation contenant de l'oxygène, où l'alcène comprend le terpinéol, le citronellol, le nérol, le linalool, le phytol, le géraniol, l'alcool périllylique, le menthol, le géranylgéraniol ou le farnésol, et où les espèces peroxydiques ou les produits de réaction provenant de l'oxydation du menthol ou de l'alcène représentent d'environ 0,001 % à environ 30 % en poids de la formulation pharmaceutique ;
d'un solvant de pénétration, où le solvant de pénétration comprend le diméthylsulfoxyde, le stérol, la lécithme, le propylène glycol ou le méthylsulfonylméthane, et où le solvant de pénétration représente d'environ 50 % à environ 99 % en poids de la formulation pharmaceutique ;
d'une teinte contenant un métal divalent ou tnvalent chélaté, où la teinte comprend la porphyrine, le rose Bengale, la chlorophylline, l'hémine, les comnes, la texaphyrine, le bleu de méthylène, l'hématoxyline, l'éosine, l'érythrosine, la lactoflavine, la teinte d'anthracène, l'hypéricine, le méthylcholanthrène, le rouge neutre, la phtalocyanine, la fluorescéine, l'eumélanine ou la phéomélanine, et où la teinte représente d'environ 0,1 % à environ 30 % en poids de la formulation pharmaceutique ; et
d'un composé d'oxydoréduction aromatique, où le composé d'oxydoréduction comprend la benzoquinone substituée ou non substituée, la naphtoquinone substituée ou non substituée ou l'anthraquinone substituée ou non substituée, et où le composé d'oxydoréduction aromatique représente d'environ 0,01 % à environ 20 % en poids de la formulation pharmaceutique,
pour la fabrication d'une formulation pharmaceutique destinée à résoudre le tissu cicatriciel provenant d'une brûlure.

2. Utilisation selon la revendication 1, dans laquelle l'alcène est sous forme liquide, dans une solution ou dans une dispersion.

3. Utilisation selon la revendication 1, dans laquelle l'alcène est contenu dans une huile fixe, un ester, un acide gras ou un éther.

4. Utilisation selon la revendication 1, dans laquelle l'agent d'oxydation contenant de l'oxygène comprend l'oxygène singulet, l'oxygène dans son état triplet, un anion superoxyde, le periodate, un radical hydroxyle, le peroxyde d'hydrogène, le peroxyde d'alkyle, le peroxyde de carbamoyle, le peroxyde de benzoyle ou l'oxygène lié à un élément de transition.

5. Utilisation selon la revendication 1, dans laquelle l'agent d'oxydation contenant de l'oxygène est de manière prédominante l'ozone.

6. Utilisation selon la revendication 1, dans laquelle le solvant de pénétration est un liquide, une membrane micellaire, un liposome, un émollient ou une vapeur.

7. Utilisation selon la revendication 1, dans laquelle le solvant de pénétration est le diméthylsulfoxyde (« DMSO »).

8. Utilisation selon la revendication 1, dans laquelle la teinte comprend la porphyrine, le rose Bengale ou un mélange de ceux-ci.

9. Utilisation selon la revendication 1, dans laquelle le métal comprend le fer.

10. Utilisation selon la revendication 1, dans laquelle le métal comprend le cuivre, le manganèse, l'étain, le magnésium ou le strontium.

11. Utilisation selon la revendication 1, comprenant en outre un donneur d'électrons.

12. Utilisation selon la revendication 11, dans laquelle le donneur d'électrons comprend l'acide ascorbique ou un sel pharmaceutique de celm-ci.

13. Utilisation d'espèces peroxydiques ou de produits de réaction provenant de l'oxydation du géraniol par un mélange d'ozone et d'oxygène ; de diméthylsulfoxyde (« DMSO ») ;
d'une teinte contenant un métal divalent ou trivalent chélaté, où la teinte comprend un mélange d'hématoporphyrine et de rose Bengale ou un mélange d'hématoporphyrine et de chlorophylline; et
de méthylnaphtoquinone, pour la fabrication d'une formulation pharmaceutique destinée à réparer le tissu cicatriciel provenant d'une brûlure.

14. Utilisation selon la revendication 13, dans laquelle le tissu cicatriciel provient d'une brûlure du patient.

15. Utilisation d'espèces peroxydiques ou de produits de réaction provenant de l'oxydation du menthol ou d'un alcène par un agent d'oxydation contenant de l'oxygène, où l'alcène comprend le terpinéol, le citronellol, le nérol, le linalool, le phytol, le géraniol, l'alcool périllylique, le menthol, le géranylgéraniol ou le farnésol, et où les espèces peroxydiques ou les produits de réaction provenant de l'oxydation du menthol ou de l'alcène représentent d'environ 0,001 % à environ 30 % en poids de la formulation pharmaceutique ;
d'un solvant de pénétration, où le solvant de pénétration comprend le diméthylsulfoxyde, le stérol, la lécithine, le propylène glycol ou le méthylsulfonylméthane, et où le solvant de pénétration représente d'environ 50 % à environ 99 % en poids de la formulation pharmaceutique ;
d'une teinte contenant un métal divalent ou trivalent chélaté, où la teinte comprend la porphyrine, le rose Bengale, la chlorophylline, l'hémine, les corrines, la texaphyrine, le bleu de méthylène, l'hématoxyline, l'éosine, l'érythrosine, la lactoflavine, la teinte d'anthracène, l'hypéricine, le méthylchlolanthrène, le rouge neutre, la phtalocyanine, la fluorescéine, l'eumélanine ou la phéomélanine, et où la teinte représente d'environ 0,1 % à environ 30 % en poids de la formulation pharmaceutique ; et
un composé d'oxydoréduction aromatique, où le composé d'oxydoréduction comprend la benzoquinone substituée ou non substituée, la naphtoquinone substituée ou non substituée ou l'anthroquinone substituée ou non substituée, et où le composé d'oxydoréduction aromatique représente d'environ 0,01 % à environ 20 % en poids de la formulation pharmaceutique, pour la fabrication d'une formulation pharmaceutique destinée à traiter une blessure provenant d'une brûlure.

16. Utilisation selon la revendication 1, dans laquelle l'alcène est sous forme liquide, dans une solution ou dans une dispersion

17. Utilisation selon la revendication 1, dans laquelle l'alcène est contenu dans une huile fixe, un ester, un acide gras ou un éther.

18. Utilisation selon la revendication 1, dans laquelle l'agent d'oxydation contenant de l'oxygène comprend l'oxygène singulet, l'oxygène dans son état triplet, un anion superoxyde, le periodate, un radical hydroxyle, le peroxyde d'hydrogène, le peroxyde d'alkyle, le peroxyde de carbamoyle, le peroxyde de benzoyle ou l'oxygène lié à un élément de transition.

19. Utilisation selon la revendication 1, dans laquelle l'agent d'oxydation contenant de l'oxygène comprend l'ozone.

20. Utilisation selon la revendication 1, dans laquelle le solvant de pénétration est un liquide, une membrane micellaire, un liposome, un émollient ou de la vapeur.

21. Utilisation selon la revendication 1, dans laquelle le solvant de pénétration est le diméthylsulfoxyde (« DMSO »).

22. Utilisation selon la revendication 1, dans laquelle la teinte comprend la porphyrine, le rose Bengale ou un mélange de ceux-ci.

23. Utilisation selon la revendication 1, dans laquelle le métal comprend le fer.

24. Utilisation selon la revendication 1, dans laquelle le métal comprend le cuivre, le manganèse, l'étain, le magnésium ou le strontium.

25. Utilisation selon la revendication 1, comprenant en outre un donneur d'électrons.

26. Utilisation selon la revendication 11, dans laquelle le donneur d'électrons comprend l'acide ascorbique ou un sel pharmaceutique de celui-ci.

27. Utilisation d'espèces peroxydiques ou de produits de réaction provenant de l'oxydation du géraniol par un mélange d'ozone et d'oxygène ;
de diméthylsulfoxyde (« DMSO ») ;
d'une teinte contenant un métal divalent ou trivalent chélaté, où la teinte comprend un mélange d'hématoporphyrine et de rose Bengale ou un mélange d'hématoporphyrine et de chlorophylline ; et
de méthylnaphtoquinone, pour la fabrication d'une formulation pharmaceutique destinée à traiter une blessure provenant d'une brûlure.
